# EUROPÄISCHE PATENTSCHRIFT

(11) **EP 0 456 138 B1**
(45) Veröffentlichungstag und Bekanntmachung des Hinweises auf die Patenterteilung: **13.03.1996**
(21) Anmeldenummer: 91107240.3
(22) Anmeldetag: 04.05.1991
(51) Int. Cl.: C12N 9/78, C12P 21/00, C12P 13/04

(54) **Peptidamidase und deren Verwendung**
Peptide-amidase and its application
Amidase peptidique et son application

(30) Priorität: 07.05.1990 DE 4014564
(43) Veröffentlichungstag der Anmeldung: 13.11.1991
(73) Patentinhaber: FORSCHUNGSZENTRUM JÜLICH GMBH, D-52425 Jülich (DE); Degussa Aktiengesellschaft, D-60311 Frankfurt (DE)
(72) Erfinder: Steinke, Dörte, W-5100 Aachen (DE); Kula, Maria-Regina, Prof., W-5162 Niederzier-Hambach (DE); Schwarz, Alexander, W-5000 Köln 60 (DE); Wandrey, Christian, Prof., W-5170 Jülich (DE)

(56) Entgegenhaltungen:
- EP-A- 0 334 358
- ANGEWANDTE CHEMIE, INTERNATIONAL EDITION Bd. 102, Nr. 10, 29 Oktober 1990, WEINHEIM DE; Seiten 1139-1140; D. STEINKE et al.
- PEPTIDES 1990, PROCEEDINGS OF THE EUROPEAN PEPTIDE SYMPOSIUM, 21st, 1990, 2. September 1990, Seiten 281-282; D. STEINKE et al.

## Beschreibung

Gegenstand der Erfindung ist ein bislang nicht bekanntes Enzym, das zur selektiven hydrolytischen Abspaltung der freien Aminogruppe am C-terminalen Ende von Peptidamiden befähigt ist, d.h. die folgende Umsetzung katalysiert: 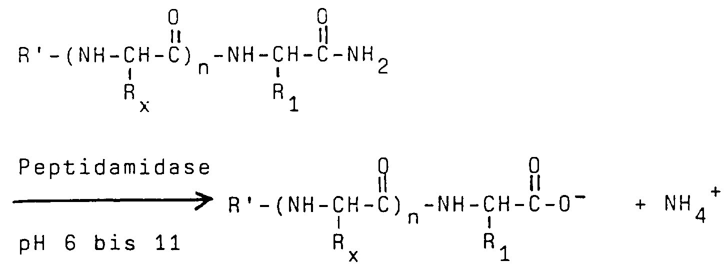 wobei:
Rₓ und R₁ für irgendeine Seitengruppe (einschließlich R = H) einer natürlichen oder nicht-natürlichen Aminosäure stehen und die Seitengruppe geschützt oder ungeschützt sein kann;
R' bei n = o eine Schutzgruppe bedeutet und bei n > o eine Schutzgruppe oder
Wasserstoff ist und
n = Null oder eine beliebige ganze Zahl ist.

Außerdem können die Wasserstoffatome in α-Stellung der einzelnen Aminosäurereste auch durch eine niedere Alkylgruppe ersetzt sein.

Die selektive Abspaltung der C-terminalen Aminogruppe von Peptidamiden ist durch eine chemische Umsetzung im allg. schwer zu erreichen, da die Peptidbindung ebenfalls einem hydrolytischen Angriff unterliegt.

Für eine enzymatische Aufspaltung der Säureamidgruppe sind zum einen Amidasen bekannt, die jedoch aufgrund ihrer α-Aminosäureamidaseaktivität nur zur Herstellung von L-Aminosäuren aus α-ungeschützten D,L-Aminosäureamiden eingesetzt werden können (DE-OS 36 29 242), Peptidamide aber nicht akzeptieren.

Andererseits kennt man Peptidasen, welche die hydrolytische Spaltung der Peptidbindungen katalysieren und von denen lediglich bekannt ist, daß sie eine gewisse Nebenaktivität zur Abspaltung der C-terminalen Aminoschutzgruppe besitzen. Ein Beispiel dafür ist die Carboxypeptidase Y, insbesondere in chemisch modifizierter Form (siehe K. Breddam, Carlsberg Res. Commun. 49 (1984) 535 -54).

Es wurde nun überraschenderweise aus dem Flavedo von Citrusfrüchten eine Peptidamidase isoliert, welche die Peptidbindungen nicht angreift, jedoch die Abspaltung der freien Aminogruppe von Peptidamiden katalysiert.

Die Erfindung betrifft mithin eine zur selektiven hydrolytischen Abspaltung der freien Aminogruppe am C-terminalen Ende von Peptidamiden befähigte Peptidamidase, gewonnen aus dem Flavedo von Citrusfrüchten.

Vorzugsweise wird die Peptidamidase aus dem Flavedo von Orangen isoliert.

Ihre selektive Wirksamkeit wird weiter unten anhand der zusammengestellten experimentellen Ergebnisse belegt. Die erfindungsgemäße Peptidamidase ist darüber hinaus auch in der Lage, die C-terminale Aminogruppe von N-terminal geschützten Aminosäureamiden abzuspalten.

In aufgereinigter Form ist die erfindungsgemäße Peptidamidase durch die folgenden Parameter gekennzeichnet:
- Abspaltung der C-terminalen Aminogruppe von Peptidamiden und N-terminal geschützten Aminosäureamiden;
- keine Spaltung von Peptidbindungen;
- optimaler pH-Wert bei 7,5 ± 1,5;
- gute Stabilität im pH-Bereich zwischen pH 6,0 und pH 9,0;
- die optimale Temperatur beträgt 30°C bei einem pH-Wert von 7,5;
- schwache Inhibierung durch Hemmstoffe von Serinproteasen, insbesondere Phenylmethansulfonylfluorid;
- das Molekulargewicht liegt bei 23000 ± 3000 da gemäß Gelelektrophorese;
   gelegentlich treten Aggregate mit einem Molekulargewicht von ca. 160 000 da auf;
- der isoelektrische Punkt liegt bei pH 9,5;

Das Enzym kann in bekannter Weise chemisch oder enzymatisch modifiziert werden bzw. in isozymer Form vorliegen.

Die erfindungsgemäße Peptidamidase zeigt weder Peptidaseaktivität noch Aminosäureamidaseaktivität, wie aus der nachfolgenden Tabelle hervorgeht, in der Aktivitäten von aufgereinigter Peptidamidase einerseits und Carboxypeptidase Y in nativer bzw. modifizierter Form einander gegenübergestellt sind:

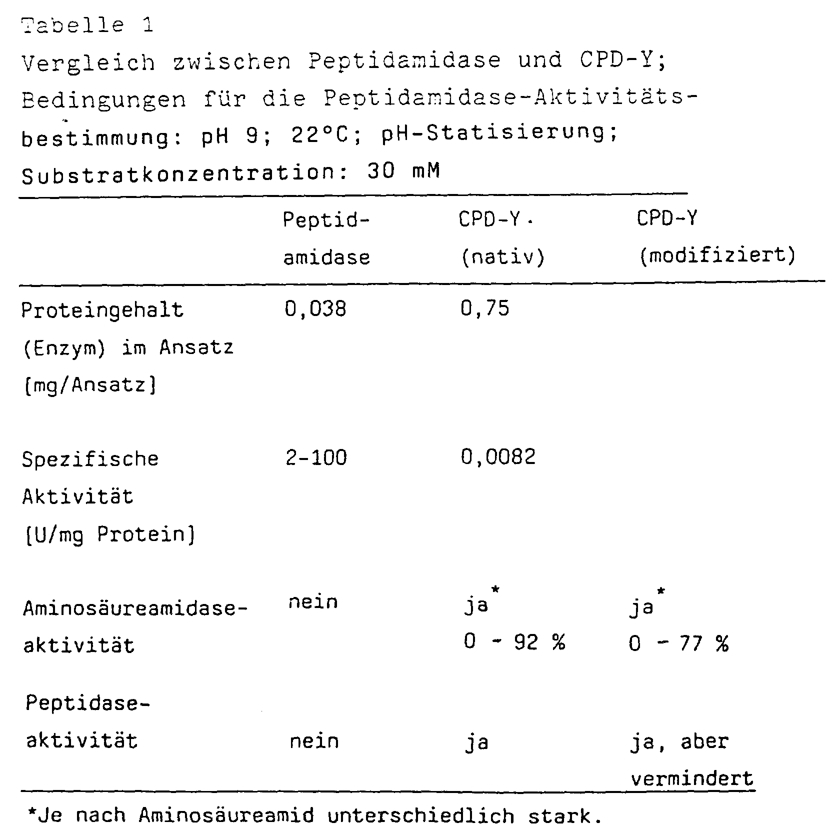

Gegenstand der Erfindung ist daher ein Verfahren zur Herstellung von Peptiden und N-terminal geschützten Aminosäuren der allgemeinen Formel 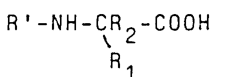 in der R' eine Schutzgruppe oder ein beliebiger peptisch oder isopeptidisch gebundener Aminosäure- oder Peptidrest ist, R₂ für Wasserstoff oder Alkyl steht und R₁ Wasserstoff oder eine beliebige, ggf. Schutzgruppen aufweisende Seitenkette bedeutet, unter enzymatischer Aufspaltung der C-terminalen Säureamidgruppe, wobei Ammoniak und die N-terminal geschützte Aminosäure oder das freie Peptid als Endprodukte entstehen.

Die erfindungsgemäße Abspaltung der C-terminalen Aminogruppe kann ohne weiteres kontinuierlich durchgeführt werden, insbesondere mit immobilisiertem Enzym, das ggf. in chemisch oder enzymatisch modifizierter Form eingesetzt wird.

Durch gentechnologische Manipulation läßt sich in bekannter Weise eine mikrobielle Produktion des Enzyms erreichen.

Die Selektivität der erfindungsgemäßen enzymatischen Reaktion ermöglicht eine wirtschaftliche Herstellung von Peptiden durch Umsetzung von ggf. N-geschützten Aminosäure- oder Peptidalkylestern mit Aminosäureamiden mit gleichzeitiger oder nachgeschalteter enzymatischer Abspaltung der Aminogruppe, wie sie im Patentanspruch 8 oder 9 angegeben ist.

Selbstverständlich ist die erfindungsgemäße enzymatische Reaktion ohne weiteres als Teilschritt einer komplexen enzymatischen Kette einzusetzen, zusammen mit anderen Enzymen, wie insbesondere Proteasen, Peptidasen, Esterasen und/oder Lipasen.

Weitere Besonderheiten ergeben sich aus der nachfolgenden Beschreibung anhand von Beispielen zur Veranschaulichung der Erfindung:

### Beispiel 1

### Isolierung und Aufarbeitung der Peptidamidase

### 1.1 Gewinnung des Enzymextraktes.

303,35 g von 17 gewaschenen Navelorangen neuer Ernte abgeriebenes Flavedo wurden in 2,3 %iger NaCℓ-Lösung unter Eiskühlung mit grobem und feinem Rührstab homogenisiert. Die Schalenreste wurden abzentrifugiert und über Papierfilter filtriert.

### 1.2 Ammoniumsulfatfällung.

Der Extrakt wurde zunächst mit festem Ammoniumsulfat bis zur 30 % Sättigung versetzt. Das Präzipitat besteht nur zu einem kleinen Teil aus Protein. Der pH der Lösung wurde nicht eingestellt. Er betrug pH 5.5. Die Hauptaktivtät befindet sich in der 30-70 % Fraktion. Das Präziptat wurde 25 min bei 10000 U/min abzentrifugiert und konzentriert in 50 mM Tris/HCl, pH 7.5 gelöst. In diesem Schritt wurde von 795 ml auf 100 ml Volumen aufkonzentriert (0.69 mg/ml Protein auf 2.94 mg/ml). Dauer der Fällung: ca.3 h.

### 1.3 Gelchromatographie mit Sephadex® G-10

In diesem Schritt wurde die Proteinlösung umgepuffert in 50 mM Tris, pH 7,5 und gleichzeitig niedrigmolekulare Bestandteile entfernt, die als leuchtend gelbe Bande erkennbar werden.
Laufbedingungen: Säule: 26,5 x 5 cm (520 ml), äquilibriert mit 50 mM Tris/HCl; pH 7,5
Flußrate: 1,66 ml/min
Probevolumen: 50 ml (147 mg Protein; 59,1 U;
Substrat : Z-Gly-Tyr-NH₂; siehe Beispiel 1.5)

Der umgepufferte Enzymextrakt (150 ml) wurde anschließend mit einer Amicon Spiralultrafiltration (YM 2000 Membran) auf 52 ml aufkonzentriert, die für die Ionenaustauschchromatographie verwendet wurden.

### 1.4 Ionenaustauschchromatographie mit Sephadex® A-50

52 ml aufkonzentrierte G-10 Fraktion wurden auf die Säule aufgepumpt. Die Peptidamidase wird mit dem Durchlauf-Proteinpeak als klare, farblose Lösung eluiert.
Laufbedingungen: Säule: 16,5 x 5 cm (324 ml)
Flußrate: 0,4 ml/min
Probevolumen: 52 ml (88,2 mg Protein) mit 25 mM Tris/HCl, pH 7,5.

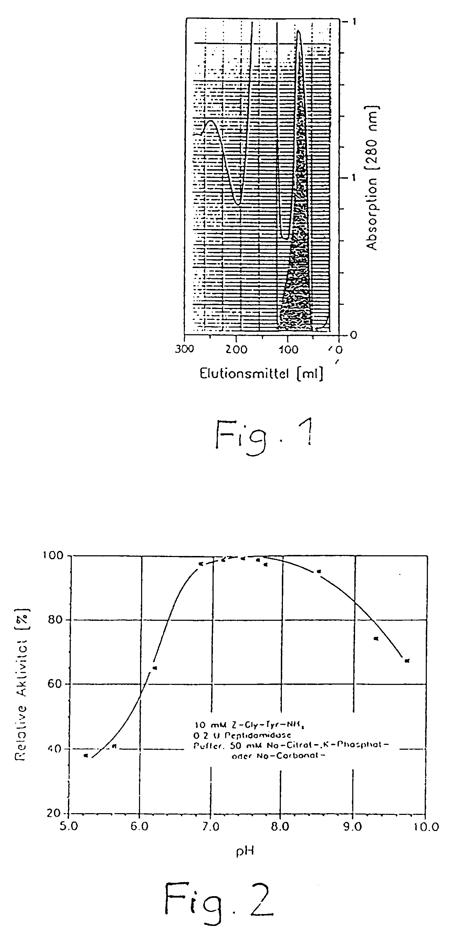

Die beigefügte Fig. 1 zeigt ein typisches Chromatogramm mit DEAE Sephadex® A-50.

In der nachfolgenden Tabelle 2 sind die Ergebnisse der Aufreinigungsschritte zusammengestellt.

**Tabelle 2**

| | Volumen | Protein | Gesamtprotein | Aktivität | Gesamtaktivität |
|---|---|---|---|---|---|
| | [ml] | [mg/ml] | [mg] | [U/mg] | [U] |
| Extrakt | 795 | 0,694 | 552 | 0,355 | 196 |
| Fraktion (NH₄)₂SO₄ | 100 | 2,94 | 294 | 0,402 | 118 |
| G-10 | 300 | 0,795 | 238 | 0,43 | 103 |
| G-10 (UF) | 100 | 1,69 | 176 | 0,59 | 103 |
| IAC A-50 | 174 | 0,16 | 27.8 | 1,65 | 45,6 |
| IAC-A-50 (UF) | 28 | 0,80 | 22.6 | 1,63 | 45.52 |

- G-10 :: Umpufferung mit Sephadex® G-10
- IAC :: Ionenaustauschchromatographie an DEAE Sephadex® A-50
- UF :: Ultrafiltration
- Fraktion :: 30 - 70 % (NH₄)₂SO₄

### 1.5 Bestimmung der Enzymaktivität

Obwohl mit anderen Substraten, z.B. H-Tyr-Arg-NH₂, wesentlich höhere Aktivitätswerte gemessen wurden, wurde Z-Gly-Tyr-NH₂ als Substrat verwendet. N-terminal freie Peptide werden von Proteasen, die sich zu Beginn der Aufreinigung im Ansatz befinden, schnell hydrolysiert, wohingegen Z-Gly-Tyr-NH₂ nicht gespalten wird. Dieses Substrat eignet sich somit als Substrat für die Aktivitätsbestimmung auch in ungereinigten Extrakten, da zudem eine vollständige Trennung mit der HPLC in 6-8 min bei 280 nm (wenig Störfaktoren) möglich ist.

### Reaktionsansatz:

| | | |
|---|---|---|
| 100 mM | Z-Gly-Tyr-NH₂ | |
| | in Wasser/Ethanol (1:1) | 0,1 ml |
| 0,8 mg/ml | Enzymlösung | 0,05 - 0,3 ml |
| 0,05 M | Tris/HCl; pH 7,5 | 0,85 - 0,6 ml |

Zu verschiedenen Zeitpunkten wurde 0,1 ml der Reaktionslösung in Eisessig (1:1) pipettiert, um die Reaktion zu stoppen. Die quantitative Bestimmung des gebildeten Z-Gly-Tyr-OH erfolgt mit der HPLC. Aus den integrierten Flächen wird mittels einer Eichkurve die gebildete Menge Z-Gly-Tyr-OH zu etwa 5 Zeitpunkten ermittelt.

Aus der Steigung, also der Zunahme von Z-Gly-Tyr-OH, ergibt sich die Reaktionsgeschwindigkeit.

Die Trennung erfolgte mit einer ODS-Hypersil 5 µ RP-18 Säule mit dem Laufmittel Tetrabutylammoniumhydrogensulfat (0,1 M) / Acetonitril (65/35) bei 0,1 ml/min.

### Retentionszeiten:

| | |
|---|---|
| Z-Gly-Tyr-NH₂ : | 4,5 (Substrat) |
| Z-Gly-Tyr-OH : | 5,9 (Produkt) |

Die daraus ermittelte Aktivität für das Substrat Z-Gly-Tyr-NH₂ liegt bei 2 - 4 U/mg Protein (gereinigtes Enzym).

Der Protein-Extrakt enthält bis vor der Ionenaustausch-Chromatographie neben der Peptidamidase als weiteres Enzym Carboxypeptidase. Diese Enzymmischung kann also ggf. für die schrittweise Synthese von Peptiden durch jeweilige Ankopplung von Aminosäureamid und Amidspaltung benutzt werden (siehe Beispiel 6).

### Beispiel 2 Charakterisierung des Enzyms

### A. Abhängigkeit der Aktivität vom pH-Wert

Die Reaktionsgeschwindigkeit der Aufspaltung der Säureamidgruppe aus der Verbindung Benzyloxycarbonylglycyltyrosinamid (Z-Gly-Tyr-NH₂) wurde in Abhängigkeit vom pH - Wert der Reaktionsmischung bestimmt.

Der Testansatz war wie folgt zusammengesetzt:

| | | |
|---|---|---|
| 100 mM | Z-Gly-Tyr-NH₂ in Wasser/Ethanol (1:1) | 0,1 ml |
| 0,17 mg/ml | Enzymlösung | 0,3 ml |
| 0,1 M | Puffer | 0,6 ml |

Es wurden vor Reaktionsbeginn verschiedene pH - Werte im Bereich von pH 4,0 - 5,5 in Citratpuffer, pH 6,0 - 8,5 in Kaliumphosphatpuffer und pH 9,0 - 10,0 in Natriumcarbonatpuffer durch Zugabe von Natriumhydroxid bzw. Salzsäure eingestellt. Das Enzym wurde in dem entsprechendem Puffer bei T = 30°C für 15 min inkubiert. Die Reaktion wurde durch Zugabe von Substrat gestartet und der pH-Wert im Reaktionsansatz erneut bestimmt. Nach 30 min, 1 h und 2 h wurden Aliquots der Reaktionsansätze mit 1:1 Eisessig gestoppt um die Enzymaktivität zu bestimmen (siehe Beispiel 1).

Das Enzym weist ein relativ breites pH Optimum von pH 6-9 auf. Bei pH-Werten um pH 10 ist noch deutliche Aktivität von 65 % des Optimums festzustellen (Fig.2).

### B. Abhängigkeit der Aktivität und Stabilität von der Reaktionstemperatur

Testansätze mit 10 mM Z-Gly-Tyr-NH₂ wurden in 50 mM Tris/HCl Puffer, pH 7,5 zwischen 11°C und 56°C vortemperiert. Die Reaktion wurde durch Zugabe von Enzymlösung gestartet. Der Reaktionsansatz war entsprechend Beispiel 2A. Für die Aktivitätsbestimmung wurden Aliquots der Reaktionsansätze nach unterschiedlicher Reaktionsdauer in gekühlten Eisessig pipettiert, mittels HPLC analysiert und aus der Steigung im linearen Bereich die Aktivität bestimmt.
Zur Bestimmung der Stabilität in Abhängigkeit von der Temperatur wurde das Enzym in Puffer bei der jeweiligen Temperatur 10, 30, 65, 115, 171, 235 und 310 min vorinkubiert. Die Reaktion wurde durch Zugabe von Substrat gestartet und die Aktivität bestimmt. Bei 25°C bis 30°C war bei den gemessenen Inkubationszeiten kein Aktivitätsverlust festzustellen. Bei 22°C (Raumtemperatur) konnte das Enzym über mehrere Tage im Enzymmembranreaktor ohne Aktivitätsverlust eingesetzt werden.

**Tabelle 3**

| Aktivität und Inaktivierungsrate der Peptidamidase in Abhängigkeit von der Temperatur (s.a.Fig.3) | | | |
|---|---|---|---|
| **Temperatur** | **Spezifische Aktivität** | **Inaktivierungsrate** | |
| **[°C]** | **[U/mg]** | **[%]** | **[%/min]** |
| 11 | 0,26 | 9,3 | |
| 22 | 0,84 | 30,4 | |
| 25 | 1,25 | 45,2 | |
| 30 | 1,83 | 66,2 | |
| 37 | 2,74 | 98,9 | 0,015 |
| 56 | 2,77 | 100 | 1,35 |

### C. Einfluß von Chelatbildnern, Metallkationen und Enzyminhibitoren auf die Peptidamidaseaktivität.

Der Einfluß folgender Enzymeffektoren auf die Peptidamidaseaktivität wurde unter Standardbedingungen (s. Beispiel 1) bestimmt. Die Inhibitorkonzentration betrug jeweils 10 mM.

Wie aus Tabelle 4 hervorgeht, wird die Aktivität lediglich durch PMSF signifikant gehemmt.

**Tabelle 4**

| Wirkung verschiedener Effektoren auf die Peptidamidaseaktivität. | |
|---|---|
| Effektor [0.01 M] | % Restaktivität |
| **Komplexbildner:** | |
| NaN₃ | 100 % |
| EDTA | 92 % |

| **Inhibitoren von PLP-Enzymen:** | |
|---|---|
| Cycloserin | 100 % |
| Semicarbazid | 102 % |

| **Inhibitoren von Serin-Hydrolasen:** | |
|---|---|
| PMSF | 55% |
| Andere: | |
| Cycloheximid | 98 % |
| Glycin | 99 % |
| Dithiotreitol | 100 % |

| **SH-Gruppenreagentien:** | |
|---|---|
| pOHMBA | 96 % |
| Jodacetamid | 99 % |
| EMI | 100 % |
| HgCl₂ | 95 % |

- pOHMB :: para - Hydroxymercuribenzoat
- EMI :: Ethylmaleiimid
- PMSF :: Phenylmethansulfonylfluorid
- EDTA :: Ethylendinitrilotrtraessigsäure

### D. Lagerungsstabilität

Die Aktivität der Peptidamidase wurde über einen Zeitraum von mehreren Monaten bei unterschiedlichen Lagerungsbedingungen gemessen.

Die Enzymlösung (gemäß 1.3) wurde folgendermaßen gelagert:
- Umgepuffert in 0,1 M Citratpuffer, sterilfiltriert, im ebenfalls sterilisiertem Glas bei 4°C (lichtgeschützt) in50 mM KPi pH 7,5
- Identische Lösung bei -20 °C ohne Zusätze eingefroren
- über G-10 Säulen (2,5 ml) kurz vor Aktivitätsmessung entsalzte 30-80% (NH₄)₂SO₄ Lösung (Puffer: 50 mM KPi, pH 7,5)

Die Reaktionsgeschwindigkeit wurde nach 1, 5, 12, 17, 38 und 124 Tagen gemessen. In diesem Zeitraum konnte in keinem der Ansätze ein Aktivitätsverlust festgestellt werden. Das Enzym kann auch im (gemäß 1.4) gereinigten Zustand mehrere Wochen (Meßdauer) ohne Aktivitätsverlust bei 4°C gelagert werden.

Das gereinigte Enzym kann lyophilisiert werden.

### E. Bestimmung des Molekulargewichts

Das Molekulargewicht wurde mit SDS-PAGE Elektrophorese nach Lämmli ermittelt. Ein Teil der Proben wurde mit 10% Mercaptoethanol versetzt, um das Molekulargewicht der Untereinheiten bestimmen zu können. Aus den Proben ohne Zusatz von Mercaptoethanol wurde das Molekulargewicht des nicht reduzierten Enzyms ermittelt.
Die Auswertung erfolgte über eine Eichgerade der Standardproteine (Boehringer Mannheim).
Bedingungen: 20 % (10 %) Trenngel, 2 % Sammelgel;
Elektrophoresekammer: Bio-Rad ProteanTM II mit Desatronic 3000/200 (Desaga, Heidelberg) power supply.
Laufbedingungen: 80 V (15 mA) im Sammelgel, 200 V (35 mA) im Trenngel, Färbung: Coomassie Blue

Das Molekulargewicht der Peptidamidase beträgt 23000 ± 3000. Bisweilen treten größere Aggregate mit einem mittleren MG von etwa 160000 D auf.

### Beispiel 3: Substratspektrum der Peptidamidase

### A. Stereospezifität des Enzyms bei verschiedenen Peptidderivaten,die D-Aminosäuren enthalten.

**Tabelle 5**

| Deamidierung von Tripeptiden, die D-Aminosäuren enthalten. | | |
|---|---|---|
| Substrat | Produkt | Umsatz [ % / 12 h] |
| H-Gly-D-Phe-Tyr-NH2 | H-Gly-D-Phe-Tyr-OH | 100 |
| H-Gly-Phe-D-Phe-NH2 | H-Gly-Phe-D-Phe-OH | 0 |
| H-Arg-Pro-D-Ala-NH₂ | H-Arg-Pro-D-Ala-OH | 0 |

- Trennbedingungen:: Säule: RP 18 0,1 ml/min
- Eluent:: TBA(Tetrabutylammoniumhydrogensulfat)/Acetonitril
Detektion bei 256 nm

### B. Spaltung von N-terminal geschützten Aminosäureamiden

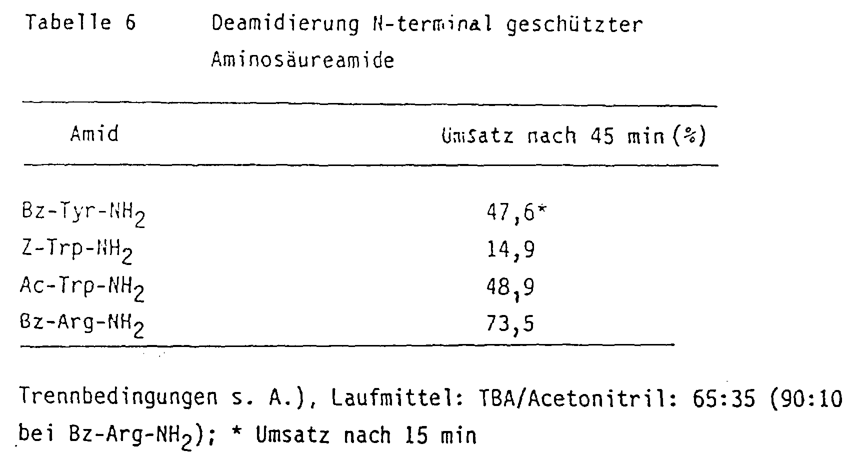

**Tabelle 6a**

| Enzymatische Umsetzung von Aminosäureamiden mit geschützter α-Aminofunktion. Reaktionsbedingungen: 10 mM Substrat; 50 µg/mlPeptidamidase; 50 mM Tris/HCl; pH 7,0, T = 30 °C. | | | | |
|---|---|---|---|---|
| Substrat | Produkt | Spez.Aktivität | Umsatz | Umsatz |
| | | [ U/mg] | [% / 28 min] | [% / 2h] |
| Bz-Tyr-NH₂ | Bz-Tyr-OH | 6,34 | 88,7 | 100 |
| Z-Trp-NH₂ | Z-Trp-OH | 0,37 | 5,16 | 23,5 |
| Ac-Trp-NH₂ | Ac-Trp-OH | 2,32 | 32,4 | 100 |
| Bz-Arg-NH₂ | Bz-Arg-OH | 4,39 | 61,6 | 100 |

### C. Spaltung N-terminal geschützter Peptid- und Aminosäureamide

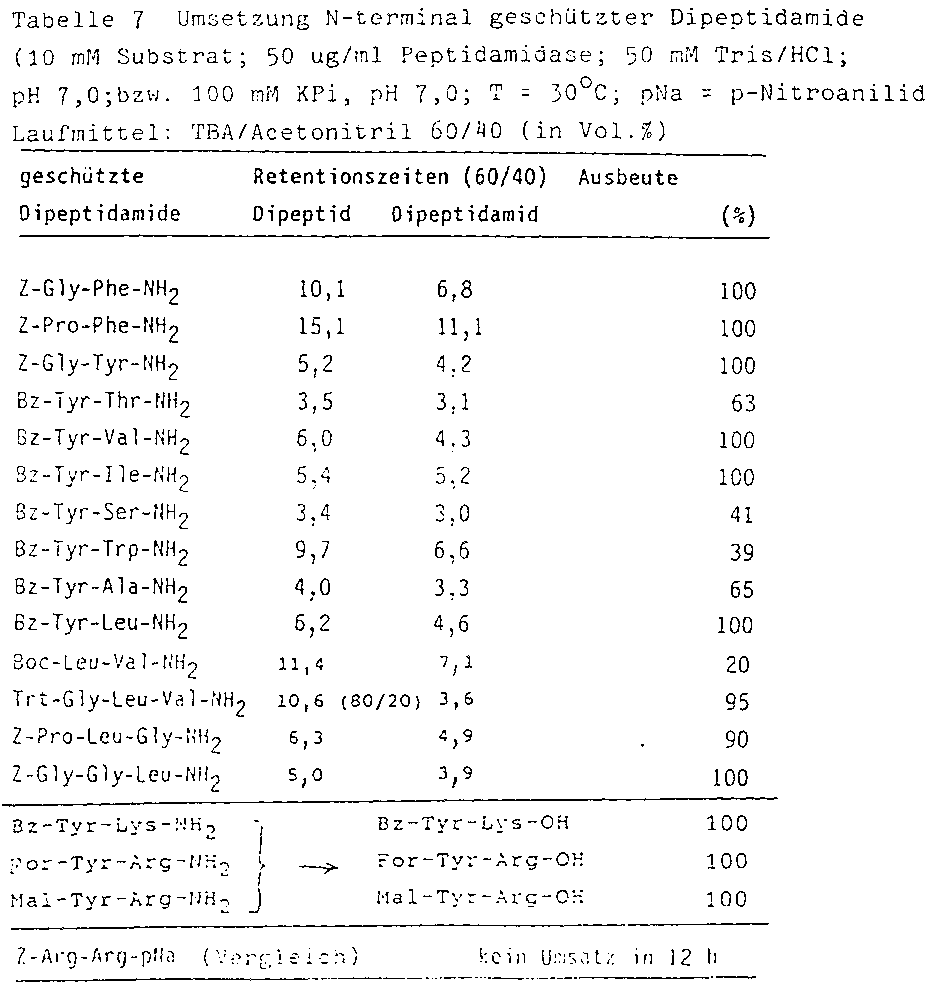

### D. Spaltung N-terminal freier Peptidamide

Bei der Deamidierung N-terminal freier Dipeptidamide (z.B. H-Tyr-Arg-NH₂) wurden erheblich höhere Aktivitäten gemessen als bei den Peptidamiden mit einer aromatischen Schutzgruppe (z.B. Z-, oder Bz-). Die Aktivitäten sind bis zu 100fach höher, als bei geschützten Peptidamiden.

**Tabelle 8**

| Enzymatische Spaltung der Säureamidgruppe von N-terminal ungeschützten Dipeptidamiden mit der Peptidamidase aus Orangenflavedo. Laufmittel (HPLC): TBA/Acetonitril 95/5 bzw. 96/4 (*) | | |
|---|---|---|
| ungeschützte Peptidamide | Peptidamid | Retentionszeiten freies Peptid |
| H-Tyr-Arg-NH₂ | 2,81 | 3,0* |
| H-Tyr-Lys-NH₂ | 2,8 | 3,0* |
| H-Tyr-Ser-NH₂ | 2,9 | 3,1* |
| H-Phe-Arg-NH₂ | 2,8 | 3,2 |
| H-Phe-Lys-NH₂ | 2,8 | 3,2 |
| H-Phe-Ser-NH₂ | 3,1 | 4,5 |
| H-Trp-Arg-NH₂ | 3,4 | 3,9 |
| H-Trp-Lys-NH₂ | 3,2 | 4,0 |
| H-Trp-Ser-NH₂ | 4,0 | 4,8 |
| H-Val-Phe-NH₂ | 11,54 | 27,3 |
| H-Asp-Phe-NH₂ | 12,12 | 12,9 |
| H-Ala-Phe-NH₂ | 4,8 | 8,1 |
| H-Arg-Met-NH₂ | 6,7 | 5,4 |
| H-Phe-Leu-NH₂ | 17,5 | 22,1 |

Die Ausbeute betrug in jedem Fall 95 - 100 %

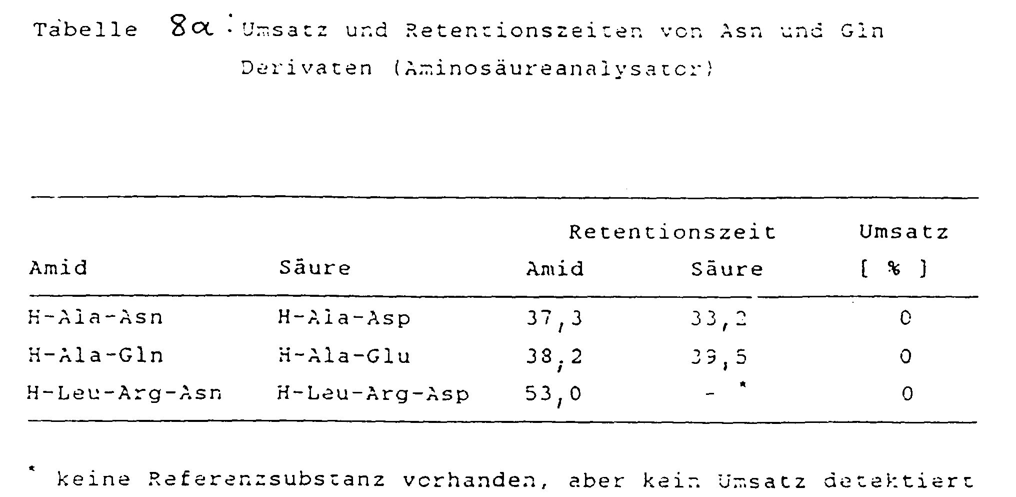

### E. Aminosäureamide

Wie aus Tabelle 9 ersichtlich, wurde keins der untersuchten Aminosäureamide deamidiert. Dies verdeutlicht die Spezifität des Enzyms für Peptidamide. Es wurde DEAE gereinigtes Enzym eingesetzt (volumetrische Aktivität: 40 U/ml für das Substrat For-Tyr-Arg-NH₂)

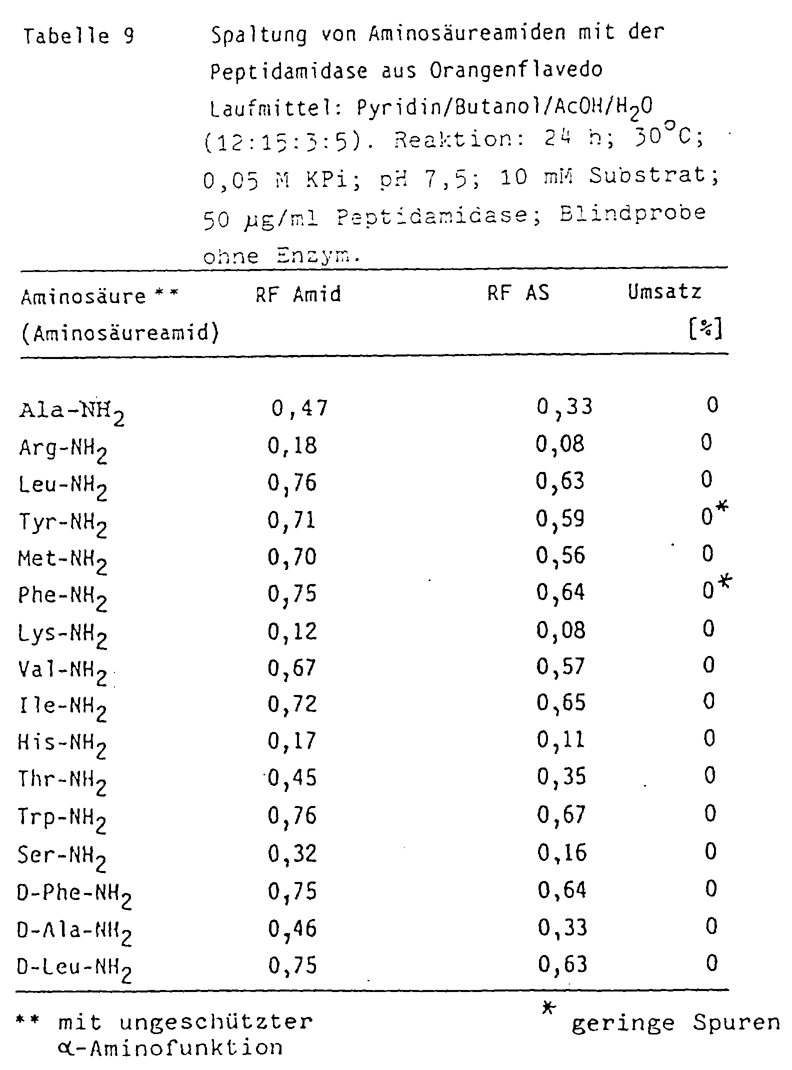

### Beispiel 4 Einfluß von organischen Lösungsmitteln auf die Enzymaktivität und -stabilität

### A. Konzentrationsabhängigkeit der Aktivität

Untersucht wurde die Abhängigkeit der Amidaseaktivität von der Konzentration wasserlöslicher Lösungsmittel wie Ethanol, Propanol, DMF, Aceton.
Ansatz: 850 µl der jeweiligen Lösungsmittel/Tris
(50 mM; pH 7,5) Mischung
100 µl Substrat (10 mM Z-Gly-Tyr-NH₂)
50 µl Amidase (40 g Protein)

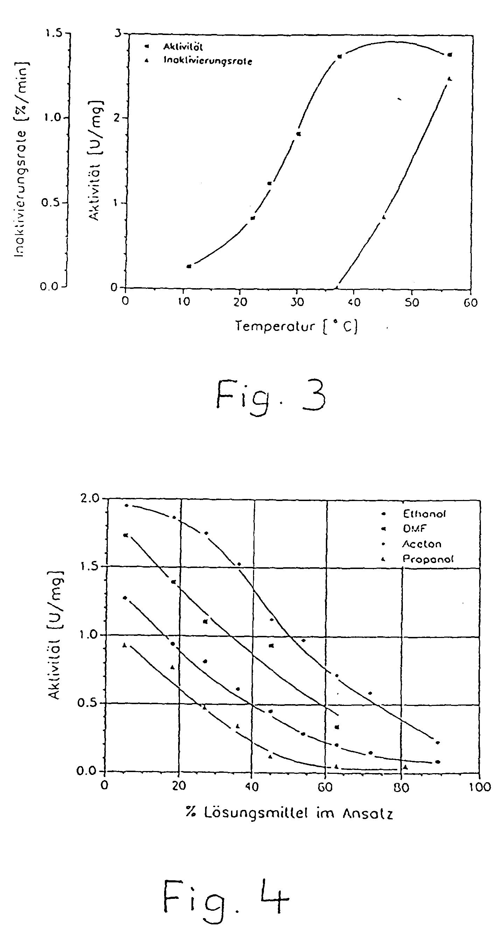

Die Reaktion wurde durch Zugabe von Enzym gestartet, um Inaktivierung des Enzyms vor Starten der Reaktion zu vermeiden. Tabelle 10 und Fig.4 zeigen die Ergebnisse.

**Tabelle 10**

| Abhängigkeit der Amidaseaktivität von der Konzentration wasserlöslicher Lösungsmittel | | | | |
|---|---|---|---|---|
| **Konz. des Lsgm.** | **Aktivität [U/mg]** | | | |
| | **Aceton** | **DMF** | **Ethanol** | **Propanol** |
| 89,1 % | 0,224 | 0 | 0,083 | 0,05 |
| 72,0 % | 0,585 | 0 | 0,149 | 0,1 |
| 63,0 % | | 0,195 | 0,204 | 0,1 |
| 54,0 % | | 0,777 | 0,284 | 0,1 |
| 45,0 % | 1,38 | 0,931 | 0,448 | 0,12 |
| 36,0 % | 1,527 | - | 0,613 | 0,342 |
| 27,0 % | 1,748 | 1,099 | 0,814 | 0,474 |
| 18,0 % | 1,863 | 1,391 | 0,938 | 0,772 |
| 5,0 % | 2,011 | 1,727 | 1,269 | 0,927 |

### B. Einfluß der Lösungsmittelkonzentration auf Km und vmax Werte

**Tabelle 11**

| Einfluß der Acetonkonzentration im Ansatz auf kinetische Parameter Substrat: Z-Gly-Tyr-NH₂, 40 µg/ml Peptidamidase, in 50 mM Tris/HCl; pH 7,5 | | |
|---|---|---|
| Konzentration (Aceton) | Km [mM] | vmax [U/mg] |
| 5 % | 0,063 | 2,93 |
| 20 % | 1,6 | 2,54 |
| 40 % | 5,5 | 2,57 |
| 60 % | 19,6 | 2,58 |

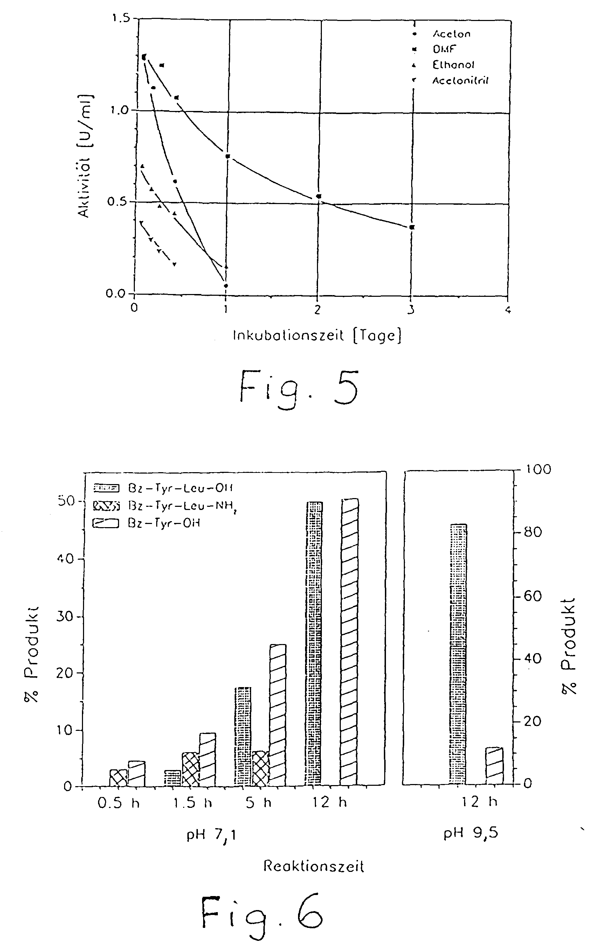

Ähnliche Ergebnisse wurden mit DMF als Lösungsmittel erhalten.
Kₘ und Vₘₐₓ zeigten keine wesentliche Änderung, wenn ein stärker polares Substrat (Bz-Arg-NH₂) verwendet wurde. C. Einfluß auf die Stabilität 
Lösungsmittel: Aceton        je 30 % Lösungsmittel
               Acetonitril   im Ansatz
               DMF           Inkubation bei 27,8 %
               Ethanol       Lösungsmittel
 10 identische Ansätze folgender Zusammensetzung:
250 µl Lösungsmittel
600 µl 50 mM Tris/HCl; pH 7,5
50 µl Enzymlösung (40 µg/Ansatz Protein) wurden bei 30 °C inkubiert. Zu verschiedenen Zeitpunkten wurde die Reaktion durch Zugabe von Substrat ( Z-Gly-Tyr-NH₂ in 100 µl Puffer/Lösungsmittel 1:1, entsprechend 10 mM/Ansatz) gestartet und die Aktivität gemessen. Die Ergebnisse sind in Fig.5 dargestellt.

Danach wird die geringste Aktivitätsabnahme in Dimethylformamid gefunden. Nach 2 Tagen Inkubation bei 27,8 % DMF und 30°C besaß das Enzym noch 28,5% der Aktivität.

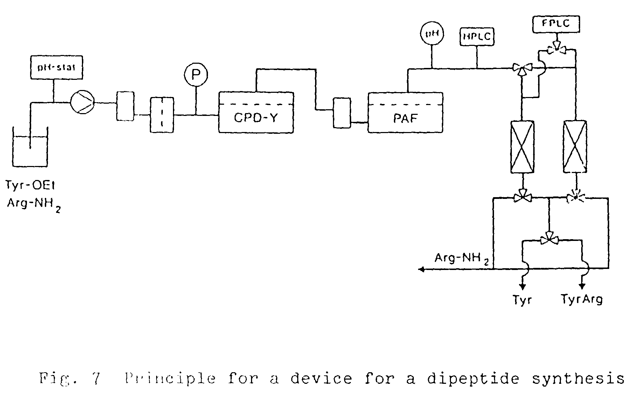

### Beispiel 5:Kontinuierliche Deamidierung von H-Tyr-Arg-NH₂ im Enzymmembranreaktor (EMR) und vereinfachte Aufarbeitung der Reaktionslösung. (Schema siehe Fig. 7)

Im EMR wurde das Verfahren kontinuierlich durchgeführt. Die Betriebsbedingungen des EMR (entsprechend kontinuierlich betriebenem Rührkesselreaktor) erlauben eine ökonomischere Ausnutzung des Enzyms, da es vor der Produktaufarbeitung durch Ultrafiltration abgetrennt wird.

Betriebsbedingungen:

| | |
|---|---|
| Tyr-Arg-NH₂: | 20 mM |
| Verweilzeit: | 30 min |
| Enzymkonzentration: | 0,15 mg/ml |
| mittlerer Umsatz: | 92 % |
| Raumzeitausbeute: | 192,5 g/(L x d) |

Die Reaktionslösung kann nun durch Anionenaustauschchromatographie aufgearbeitet werden. Hierbei wird restliches Peptidamid nicht gebunden und kann rezykliert werden, während das deamidierte Peptid gebunden und durch einen Salzgradienten eluiert wird.
- Säule:: 2,4 x 10 cm
- Säulenmaterial:: Dowex 1 x 4; 200-400 mesh
- Elutionsmittel:: Tris/HCl; pH = 9,0;
Gradient 0 - 40 % 1 M NaCl

Das Dipeptid H-Tyr-Arg-OH wird zwischen 6 und 9 % einer 1 molarer NaCl-Lösung eluiert.

### Beispiel 6: Verwendung der Peptidamidase zur simultanen Abspaltung der Amidgruppe vom Peptidamid bei gleichzeitiger enzymatischer Peptidsynthese.

Verwendet wurde ein Enzympräparat, das sowohl Carboxypeptidase C (Zuber,H., Hoppe Seyler's Z.Phys.Chem. 1968, 349, 1337-1352) für die enzymatische Synthese des Dipeptids Bz-Tyr-Leu-NH₂ (Steinke, D., Kula, M.R., Enz. Microb. Technol., 1990, Seiten 836 - 40), als auch die erfindungsgemäße Peptidamidase (PAF) enthielt. Man erhält so das C-terminal ungeschützte Dipeptid Bz-Tyr-Leu-OH (Fig. 6).
Reaktionsansatz:
36 mM Bz-Tyr-OEt
225 mM H-Leu-NH₂
0.2 M Natriumphosphatpuffer, pH 7.1
300 µl Enzympräparat (0,37 mg Protein/Ansatz)
Die gebildeten Produkte wurden mit HPLC und Aminosäureanalyse identifiziert. Das Ergebnis ist in Fig.6 dargestellt.

In der nachfolgenden Tabelle 12 sind weitere Untersuchungsergebnisse zusammengefaßt.

**Tabelle 12**

| Enzymatische Umsetzung von Peptidamiden mit einer Kettenlänge von ≥ 3 Aminosäuren. Reaktionsbedingungen: 10 mM Substrat, 50 µg/mlPeptidamidase, 50 mM Tris/HCl, pH 7.0, T = 30 °C. | | |
|---|---|---|
| Substrat | Produkt | Umsatz [ % / 6 h] |
| Trt-Gly-Leu-Val-NH2 | Trt-Gly-Leu-Val-OH | 100 |
| Z-Pro-Leu-Gly-NH2 | H-Pro-Leu-Gly-OH | 100 |
| Z-Gly-Gly-Leu-NH2 | Z-Gly-Gly-Leu-OH | 100 |
| Z-Pro-Arg-Gly-NH₂ | Z-Pro-Arg-Gly-OH | 100 |
| H-Pro-Arg-Gly-NH₂ | H-Pro-Arg-Gly-OH | 100 |
| VP-Antagonist | VP-Antagonist (freie Säure | 100 |
| Boc-Cys(Bzl)-Pro-Arg-Gly-NH₂ | Boc-Cys-(Bzl)-Pro-Arg-Gly-OH | 90 |
| H-Tyr-Gly-Gly-Phe-Leu-NH₂ | H-Tyr-Gly-Gly-Phe-Leu-OH | 100 |
| LHRH | LHRH (freie Säure) | 90 |
| Z-Val-Gly-Ala-Pro-NH₂ | Z-Val-Gly-Ala-Pro-OH | 0 |

- VP-Antagonist:: Vasopressin Antagonist (9 Aminosäuren mit interner Disulfitbrücke)
- H-Tyr-Gly-Gly-Phe-Leu-NH₂:: Leu-Enkephalin
- LHRH:: Luteinizing Hormone Releasing Factor

### Beispiel 7 Stabilität bei verschiedenen pH Werten

Zur Stabilitätsuntersuchung wurden 100 µl der Enzymlösung (Konzentration 0,172 mg/ml) und 100 µl 10 mM Ammoniumcarbonatpuffer vermischt und bei verschiedenen pH werten zwischen pH 6 und pH 9 bei Raumtemperatur inkubiert. Zu verschiedenen Zeitpunkten wurden Aliquots (10 µl) gezogen und die Aktivität des Enzyms bei dem Substrat H-Tyr-Arg-NH₂ gemessen. Über den Beobachtungszeitraum von 190 Std. hinweg wurde keine merkliche Abnahme der Aktivität registriert.

| | | |
|---|---|---|
| Assay: | Substrat: | 19 mM H-Tyr-Arg-NH₂ |
| | pH Wert: | 7,5 |
| | Volumen: | 20 µl |
| | Enzym: | 0,0086 mg/ml |
| | Inkubationszeit: | 30 min |

### Beispiel 8 Einfluß verschiedener Materialien auf die Stabilität.

Zur Untersuchung der Materialverträglichkeit wurden 100 µl Enzymlösung (Konzentration 0,172 mg/ml) und 100 µl 10 mM Ammoniumcarbonatpuffer miteinander vermischt. Hierzu wurde ein Stück des zu untersuchenden Materials gegeben und bei Raumtemperatur und pH 7,5 inkubiert. Zu verschiedenen Zeitpunkten wurden Proben gezogen und die Aktivität des Enzyms untersucht. Die Messsung der Enzymaktivität erfolgte wie in Beispiel 1.5 beschrieben.

Untersucht wurden Polypropylen, Teflon, Kautschuk (O-Ring), Silikon, V4A-Stahl und Plexiglas: Es wurde keine merkliche Abnahme der Aktivität beobachtet. Wesentliche Unterschiede wurden nicht festgestellt.

Die erfindungsgemäße Abspaltung der freien Aminogruppe ist auch für die NH₂-Abtrennung bei Peptidamiden brauchbar, die nach herkömmlichen chemischen Methoden, z.B. in Merrifield Solid Phase Synthesis, auf besonders einfache Weise erhalten werden.

## Patentansprüche

1. Zur selektiven hydrolytischen Abspaltung der freien Aminogruppe am C-terminalen Ende von Peptidamiden befähigte aufgereinigte Peptidamidase,
**gekennzeichnet durch**
folgende Parameter
- Abspaltung der C-terminalen Aminogruppe von Peptidamiden und N-terminal geschützten Aminosäureamiden;
- keine Spaltung von Peptidbindungen;
- optimaler pH-Wert bei 7,5 ± 1,5;
- gute Stabilität im pH-Bereich zwischen pH 6,0 und pH 9,0;
- die optimale Temperatur beträgt 30°C bei einem pH-Wert von 7,5;
- schwache Inhibierung durch Hemmstoffe von Serinproteasen, insbesondere Phenylmethansulfonylfluorid;
- das Molekulargewicht liegt bei 23 000 ± 3 000 da gemäß Gelelektrophorese;
- der isoelektrische Punkt liegt bei pH 9,5 und ihre isozymen Formen.

2. Peptidamidase nach Anspruch 1,
gewonnen aus dem Flavedo von Citrusfrüchten.

3. Peptidamidase nach Anspruch 2,
gewonnen aus dem Flavedo von Orangen.

4. Verfahren zur Herstellung von Peptiden und N-terminal geschützten Aminosäuren der allgemeinen Formel 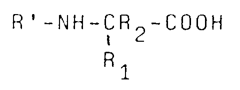 in der R' eine Schutzgruppe oder ein beliebiger peptidisch oder isopeptidisch gebundener Aminosäure- oder Peptidrest ist R₂ für Waserstoff oder Alkyl steht und R₁ Wasserstoff oder eine beliebige Seitenkette bedeutet, unter Abspaltung einer C-terminalen Aminogruppe von einem Peptidamid oder einem N-terminal geschützten Aminosäureamid,
**dadurch gekennzeichnet**,
daß man die Abspaltung enzymatisch mittels Peptidamidase nach einem der Ansprüche 1 bis 3 durchführt.

5. Verfahren nach Anspruch 4,
**dadurch gekennzeichnet**,
daß R₁ eine Schutzgruppen enthaltende Seitenkette bedeutet.

6. Verfahren nach Anspruch 4 oder 5,
**dadurch gekennzeichnet,**
daß man die enzymatische Reaktion kontinuierlich durchführt.

7. Verfahren nach einem der Ansprüche 4 bis 6,
**dadurch gekennzeichnet**,
daß man die Desamidierung als Verfahrensschritt einer gekoppelten Umsetzung mit einem Enzymsystem durchführt, das Proteasen, Peptidasen, Esterasen und/oder Lipasen umfaßt.

8. Verfahren zur Herstellung von Peptiden nach einem der Ansprüche 4 bis 7 durch enzymatische Umsetzung von ggf. N-geschützten Aminosäurealkylestern bzw. ggf. N-geschützten Peptidalkylestern mit Aminosäureamiden in wäßriger Phase oder wäßrig-organischem Milieu, in Gegenwart eines die peptidische Verknüpfung herbeiführenden Enzyms und enzymatischer Abspaltung der Amidschutzgruppe,
**dadurch gekennzeichnet**,
daß man die Synthese kontinuierlich ablaufen läßt und das Peptidamid mittels der Peptidamidase enzymatisch zum Peptid hydrolysiert und daß man schließlich das Peptid aufgrund seiner Ladung vom Reaktionsgemisch abtrennt und das Aminosäureamid zurückführt.

9. Verfahren nach Anspruch 8,
**dadurch gekennzeichnet**,
daß man das Peptid auf einem Anionenaustauscher vom Reaktionsgemisch abtrennt und das Peptid chromatographisch vom Anionenaustauscher eluiert und ggf. in das Salz oder das Hydrat überführt.

## Claims

1. Purified peptide amidase which is able to eliminate the free amino group at the C-terminal end of peptide amides hydrolytically and in a selective manner, characterized by the following parameters:
- elimination of the C-terminal amino group of peptide amides and N-terminally protected amino acid amides;
- no cleavage of peptide bonds;
- optimum pH at 7.5 ± 1.5;
- good stability in the pH range between pH 6.0 and pH 9.0;
- the optimum temperature is 30°C at a pH of 7.5;
- weak inhibition by inhibitors of serine proteases, in particular phenylmethanesulphonyl fluoride;
- the molecular weight is 23,000 ± 3000 da according to gel electrophoresis;
- the isoelectric point is pH 9.5, and its isozymic forms.

2. Peptide amidase according to Claim 1, obtained from the flavedo of citrus fruits.

3. Peptide amidase according to Claim 2, obtained from the flavedo of oranges.

4. Process for preparing peptides and N-terminally protected amino acids of the general formula 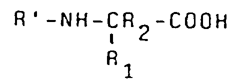 in which R' is a protecting group or an arbitrary, peptidically or isopeptidically bound amino acid residue or peptide residue R₂ represents hyrogen or alkyl, and R₁ denotes hydrogen or an arbitrary side chain, involving the elimination of a C-terminal amino group of a peptide amide or a N-terminally protected amino acid amide, characterized in that the elimination is carried out enzymically using peptide amidase according to one of Claims 1 to 3.

5. Process according to Claim 4, characterized in that R₁ denotes a side chain which contains protecting groups.

6. Process according to Claim 4 or 5, characterized in that the enzymic reaction is carried out continuously.

7. Process according to one of Claims 4 to 6, characterized in that the deamidation is carried out as a procedural step in a coupled conversion using an enzyme system which comprises proteases, peptidases, esterases and/or lipases.

8. Process for preparing peptides according to one of Claims 4 to 7 by the enzymic reaction of optionally N-protected amino acid alkyl esters or optionally N-protected peptide alkyl esters with amino acid amides in aqueous phase or aqueous/organic medium, in the presence of an enzyme which brings about peptidic linkage, and with enzymic elimination of the amide protecting group, characterized in that the synthesis is allowed to proceed continuously and the peptide amide is hydrolysed enzymically to the peptide by the peptide amidase, and in that, finally, the peptide is separated from the reaction mixture owing to its charge and the amino acid amide is returned.

9. Process according to Claim 8, characterized in that the peptide is separated from the reaction mixture on an anion exchanger and the peptide is eluted chromatographically from the anion exchanger and, where appropriate, converted into the salt or the hydrate.

## Revendications

1. Peptide-amidase purifiée capable de provoquer la séparation hydrolytique sélective du groupe amino libre à l'extrémité C-terminale d'amides peptidiques, caractérisée par les paramètres suivants :
- séparation du groupe amino C-terminal d'amides peptidiques et d'amides d'amino-acides protégés à l'extrémité N-terminale ;
- pas de séparation des liaisons peptidiques ;
- valeur optimale de pH à 7,5 ± 1,5 ;
- bonne stabilité dans la plage de pH comprise entre pH 6,0 et pH 9,0 ;
- la température optimale est de 30°C à pH 7,5 ;
- faible inhibition des sérine-protéases, en particulier du fluorure de phénylméthanesulfonyle, par des substances inhibitrices ;
- le poids moléculaire est de 23 000 ± 3 000 daltons d'après l'électrophorèse sur gel ;
- le point isoélectrique est à pH 9,5 ; ainsi que ses formes isozymes.

2. Peptide-amidase selon la revendication 1, obtenue à partir de Flavedo (partie extérieure de l'écorce du fruit) d'agrumes.

3. Peptide-amidase selon la revendication 1, obtenue à partir de Flavedo d'oranges.

4. Procédé de préparation de peptides et d'amino-acides protégés en position N-terminale et répondant à la formule générale 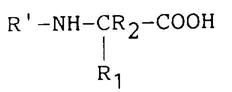 où R' est un groupe protecteur ou un reste d'amine-acide ou peptidique quelconque à liaison peptidique ou isopeptidique, R₂ représente l'hydrogène ou un groupe alkyle, et R₁ signifie l'hydrogène ou une chaîne latérale quelconque, avec séparation d'un groupe amino C-terminal et d'un amide peptidique ou d'un amide d'amino-acide protégé en position N-terminale,
caractérisé en ce qu'on effectue la séparation par voie enzymatique au moyen d'une peptide-amidase selon l'une des revendications 1 à 3.

5. Procédé selon la revendication 4, caractérisé en ce que R₁ signifie une chaîne latérale contenant des groupes protecteurs.

6. Procédé selon la revendication 4 ou 5, caractérisé en ce qu'on effectue la réaction enzymatique en continu.

7. Procédé selon l'une des revendications 4 à 6, caractérisé en ce qu'on effectue la désamidation comme une étape de procédé d'une réaction couplée avec un système enzymatique qui comprend des protéases, des peptidases, des estérases et/ou des lipases.

8. Procédé de préparation de peptides selon l'une des revendications 4 à 7 par réaction enzymatique d'alkylates d'amino-acides éventuellement N-protégés ou d'alkylates peptidiques éventuellement N-protégés, avec des amides d'amino-acides en phase aqueuse ou dans un milieu aqueux organique, en présence d'une enzyme provoquant la liaison peptidique, et séparation enzymatique du groupe protecteur de l'amide, caractérisé en ce qu'on laisse la synthèse se dérouler de manière continue, on hydrolyse l'amide peptidique en peptide par voie enzymatique au moyen de la peptide-amidase, et qu'enfin, on sépare le peptide du mélange de réaction sur la base de sa charge et on ramène l'amide d'amino-acide.

9. Procédé selon la revendication 8, caractérisé en ce qu'on sépare le peptide du mélange de réaction sur un échangeur d'anions, on élue le peptide de l'échangeur d'anions par chromatographie et on le transforme éventuellement en sel ou en hydrate.
